Europäisches Patentamt

(19) European Patent Office (11) Publication number: **0 168 244**

Office européen des brevets **A2**

(12) **EUROPEAN PATENT APPLICATION**

(21) Application number: 85304910.4 (51) Int. Cl.⁴: **A 61 B 17/10**

(22) Date of filing: 10.07.85

(30) Priority: 10.07.84 GB 8417562
05.06.85 US 741483

(43) Date of publication of application:
15.01.86 Bulletin 86/3

(84) Designated Contracting States:
AT BE CH DE FR GB IT LI LU NL SE

(71) Applicant: SYNCARE INC.
P.O. Box 648
Lebanon New Jersey 08833(US)

(72) Inventor: Taylor, James
9 Maple Grove
Droitwich Worcestershire WR9 7QF(GB)

(74) Representative: Milhench, Howard Leslie et al,
R.G.C. Jenkins & Co. 12-15, Fetter Lane
London EC4A 1PL(GB)

(54) Surgical stapling device.

(57) A surgical stapling device utilizes staples each having a pair of parallel limbs bridges by a straight cross-piece. As the limbs are implanted by the device on opposite sides of a skin incision, they are bent inwardly towards each other below the skin surface to close the incision and the cross-piece is bent to create an arched air gap above the skin surface to allow space for post-operative swelling and to facilitate subsequent removal of the staple. In the device, a row of staples straddling a rail (20a) are advanced toward a metal mandrel (20b) projecting from the front end of the rail into an outlet (19). The mandrel (20b), which has an arched formation, is flanked by a pair of abutments (29) on the front end of the rail (20a) whereby the foremost staple in the advancing row is deposited onto the mandrel (20b). Cooperating with the mandrel (20b) is a plate-like former (12) whose leading edge has a recess therein leading to an inner arched section (13) which generally conforms to the arched mandrel. When the device is actuated, the leading edge of the former (12) which is normally retracted from the mandrel (20b), advances towards the mandrel (20b), the former (12) sliding along the end abutments (29) of the rail (20a) and causing the staple engaged thereby to emerge from the outlet (19), in the course of which movement the staple is implanted in the skin and is formed on the mandrel to assume the desired configuration.

Fig.2.

-1-

## SURGICAL STAPLING DEVICE

## BACKGROUND OF INVENTION

### Field of Invention:

This invention relates generally to surgical stapling devices.

### Status of Prior Art:

In surgical procedures, the conventional skin closure technique involves a needle and a suture thread. Such suturing leaves crosshatching across the scar line as a result of pressure applied to the region during the healing process. It has therefore become cosmetically desirable to achieve an effective wound closure with the minimum amount of crosshatching.

The use of surgical stapling devices has been proposed as an alternative to needle and thread suturing. These devices make use of standard staples having a pair of parallel limbs bridged by a straight cross-piece, the limbs being implanted in the skin on opposite sides of the incision and being bent inwardly toward each other to form a loop closing the incision.

Surgeons have been making increasing use of surgical stapling devices rather than conventional thread sutures in that it is a less difficult and much faster procedure. The use of surgical staples reduces the time required for suturing and hence the length of time a patient must be maintained under anaesthesia.

In the course of healing, all wounds swell due to oedema, thereby filling the air gap between the wound and the straight cross-piece of the staple and giving rise to crosshatching. Thus known types of surgical staplers do not overcome the crosshatching problem and its adverse effects. Moreover, because the air gap is filled with swollen tissue, it becomes difficult when the staples are later to be removed from the wound, to insert the staple remover under the staple cross-piece without inflicting tissue trauma and thereby causing discomfort to the patient.

## SUMMARY OF INVENTION

In view of the foregoing, the main object of this invention is to provide an improved surgical stapling device for closing incisions made in the skin.

According to a first aspect of this invention therefore there is provided a surgical stapling device which is adapted to implant standard surgical staples into the skin and so to form the staple during implantation as to raise a portion thereof above the skin surface in order to create an arched air gap between the skin surface and the back of the staple.

Utilization of such a device according to the invention obtains the following advantages, namely, the raised staple allows space for oedema or post-operative swelling, it lessens scarring and crosshatching, for it does not strangulate the skin tissue, and it facilitates subsequent removal of the staple in that it permits easy insertion of a staple remover under the staple cross-piece, thereby minimizing tissue trauma and discomfort to the patient.

More particularly in accordance with this first aspect of the present invention, there is provided a skin-closure device comprising a housing; a handle movably mounted relative to the housing; a former mounted in the housing so as to be movable upon movement of the handle in one direction from an inoperative limit position to an operative limit position; a mandrel disposed adjacent an outlet opening in the housing; means for supplying skin-closure staples to a location on the mandrel which is in the path of movement of the former in said one direction; said former and said mandrel being shaped so that when the device is used to close an incision in the skin, the cross-piece of the staple is arched above the incision so as to ensure that there is an air gap provided between the incision and the closure element.

Conveniently, the cross-piece of the staple is arcuately arched, though other formations are possible within the ambit of the invention.

It is desirable that flat staples are used for skin-closure as a twisted staple may produce uneven forces when holding an incision closed and so cause uneven scar tissue formation. During the formation of the staple, shear forces are applied to the staple by the former and so that staple will rotate resulting in a twisted finished staple unless prevented from doing so. Most conventional paper staplers prevent twisting by effecting staple formation in a slot which is only marginally larger than the staple thickness. However, this method is unsuitable for a device which is used for skin-closure as it is necessary to be able to remove the mandrel of the closure device from between the cross-piece of the staple and the skin after the staple is in position in the skin.

Consequently, it is another feature of the present invention to provide means for preventing twisting of the staple during formation whilst allowing removal of the mandrel of the forming device after the staple is in position in the skin.

According to a second aspect of the present invention, therefore there is provided a skin-closure device comprising a housing; a handle movably mounted relative to the housing; a former mounted in the housing so as to be movable upon movement of the handle in one direction from an inoperative limit position to an operative limit position; a mandrel disposed adjacent an outlet opening in the housing; means for supplying skin-closure staples to a location on the mandrel which is in the path of movement of the former in said one direction, the mandrel having a free end on the opposite side of said location to the staple supplying means such as to enable the mandrel to be detached from a formed staple in use, and abutment means disposed between said location and said staple supply means and extending away from the mandrel on the opposite side thereof to said former so as to be abutted by a staple during forming thereof whereby twisting of the staple during forming is prevented.

It is preferred for the abutment means to be defined by an end surface of a rail which forms part of the means for supplying skin-closure staples in which said part of the mandrel is preferably embedded.

It is particularly preferred that the end of the rail forming part of the means for supplying skin-closure staples is spaced from the housing by a distance which is marginally less than the thickness of a staple such that the part-formed staple will be retained in position on the mandrel by friction between

0168244

-6-

the staple and the housing and end of the rail. The mandrel is capable of being removed when the staple is fully formed as the staple is then of a shape to pass through the opening in the housing.

Yet another object of the invention is to provide a device of simple, yet efficient and reliable construction, whereby the surgical stapling device may be made in low-cost disposable form.

According to a third aspect of the present invention therefore there is provided a skin-closure device comprising a housing; a handle pivotally mounted relative to the housing; a former mounted in the housing so as to be movable upon pivotal movement of the handle in one direction from an inoperative limit position to an operative limit position; a first resilient means biasing the former in the opposite direction; a mandrel disposed adjacent an outlet opening in the housing; means for supplying closure elements to a location on the mandrel which is in the path of movement of the former in said one direction; and a detent and release mechanism which retains said former in at least one intermediate position against movement in said opposite direction when the handle is released during movement of said former in said one direction but which permits said former to be moved in said opposite direction into its inoperative limit position by said first resilient means upon release of the handle when the former has reached its operative limit position.

With such a detent and release mechanism, the operator can carefully monitor the forming operation of a closure member.

Preferably, the first resilient means is arranged to act on, and is most preferably integral with, the handle and thereby on said former and, preferably also, said detent and release mechanism is similarly arranged.

Conveniently, the detent and release mechanism includes a first part which is mounted for movement with the handle, a second part which is fixed relative to said housing, and a second resilient means biasing at least one of said first and second parts so that the second part is arranged in the path of movement of the first part, each of said first and second parts having a ribbed surface portion and a relatively smooth surface portion which are mutually inclined, the ribbed surface portions of the first and second parts being arranged (a) so that, in said at least one intermediate position of said former, the ribbed surface portions are urged into mutual engagement by said second

-8-

resilient means and (b) so that they are out of mutual engagement when said former is in said operative limit position whereby said second resilient means effects relative movement of the first and second parts to enable the relatively smooth surface portions of the first and second parts to come into mutual contact after subsequent release of the handle and thereby permit relatively unrestricted movement of the first part past the second part during movement of said former from its operative limit position to its inoperative position under the action of the first resilient means.

Described hereinafter is an exemplary surgical stapling device according to the invention which is adapted to be loaded with staples each having a pair of parallel limbs bridged by a straight cross-piece. In operation, as the limbs are implanted by the device on opposite sides of a skin incision, they are bent inwardly toward each other below the skin surface to close the incision, the cross-piece being bent to create an arched air gap above the skin surface to allow space for post-operative swelling and to facilitate subsequent removal of the staple. In the device, a row of staples straddling a rail are advanced toward a metal mandrel projecting from the front end of the rail into an outlet. The mandrel, which has an arched formation, is flanked by a pair of abutments on the front end of the rail whereby the foremost staple in the advancing row is deposited onto the mandrel. Cooperating with the mandrel is a plate-like former whose leading edge has a recess therein leading to an inner arched section which is shaped generally to conform to the arched mandrel. When the device is actuated, the leading edge of the former which is normally retracted from the mandrel, advances toward the mandrel, the former sliding along the end abutments of the rail and causing the staple engaged thereby to emerge from the outlet, in the course of which movement the staple is implanted in the skin and is formed on the mandrel to assume the desired configuration.

## OUTLINE OF DRAWINGS

For a better understanding of the invention as well as other objects and further features thereof, reference is made to the following detailed description to be read in conjuction with the accompanying drawings, wherein:

Fig. 1 is a side view of an exemplary surgical stapling device according to the invention;

Fig. 2 is a schematic illustration of the device of Fig. 1 shown with a housing part removed;

Fig. 3 is a sectional view on a larger scale along the plane indicated by the line A-A in Fig. 2;

Fig. 4 is a diagrammatic view of a skin incision closed by a conventional staple;

Fig. 5 is a diagrammatic view of a skin incision closed by a staple formed by a device according to the invention;

Fig. 6 is an end view of a preferred former included in the device of Figs. 1 to 3;

Fig. 7 is an end view of an alternative configuration of former and a mandrel usable in a device according to the invention;

Fig. 8 is a side view of the mandrel and rail included in the embodiment of the invention shown in Fig. 2;

Figs. 9, 10 and 11 are side, plan and end views, respectively, of the mandrel included in the embodiment of the invention shown in Fig. 2; and

Fig. 12 is an end view of the embodiment of the invention shown in Fig.1.

<u>DESCRIPTION OF INVENTION</u>

<u>Structure</u>:

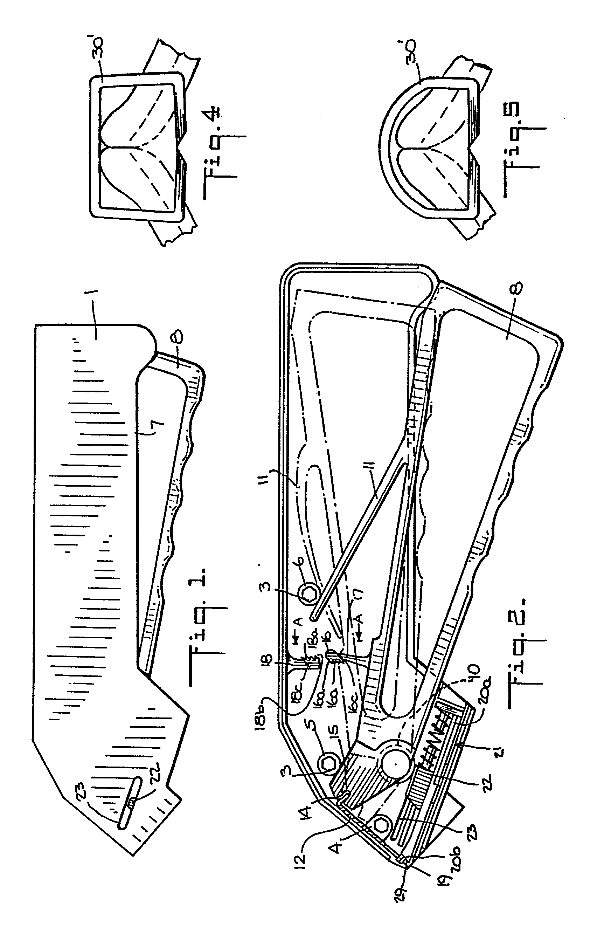

Referring now to Figs. 1, 2 and 3 in the drawings, a skin-closure surgical stapling device in accordance with the invention comprises a housing formed of a complementary pair of injection-moulded plastics parts 1 and 2. These housing parts are joined together by deformable platics fasteners 3 which engage bosses 4, 5 and 6 (shown only in respect to housing part 2) and by welding along the mutually interengaging peripheral walls. Housing 1-2 is slotted along its underside 7 to receive a handle 8 formed of an injection-moulded plastics material. Handle 8 is provided with integrally moulded pivots which are received in respective short sleeves 10 moulded integrally with housing parts 1 and 2.

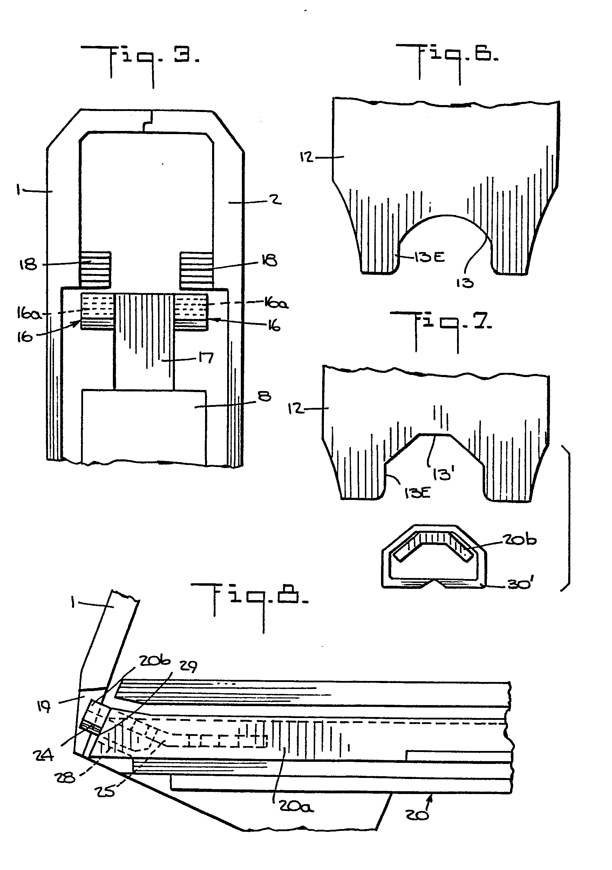

A spring arm 11 which may be of leaf spring metal or plastics material and can be moulded integrally with the handle 8, extends from an upper edge of handle 8 and abuts boss 6, the spring acting to urge the handle outwardly to the extended position illustrated in full line in Fig. 2. In this position, a nose portion of handle 8 abuts boss 5 to limit outward movement of the handle relative to the housing under the action of spring 11. The provision of a plastics spring arm which is integral with the handle reduces the number of components, thereby simplifying assembly and also reduces friction in movement of the handle compared with the use of a metal spring blade.

A former 12 defined by a sheet metal plate is so disposed in the housing as to be slidable relative thereto between an upper, inoperative limit position and a lower, operative limit position. Former 12 lies between boss 4 and the adjacent peripheral end walls of the respective housing parts 1 and 2 which define a guide for sliding movement of the former.

Boss 4 has a surface facing former 12 which is spaced from the adjacent wall of housing part 1, 2 by a distance which is only marginally greater than the thickness of the former. The width of former 12 is marginally less than the distance between the internal side faces of the housing so that the two housing parts 1 and 2 define a guide to ensure that former 12 does not become misaligned during its sliding movement.

At its lower or leading edge (as viewed in Fig. 6), former 12 is provided with a recess having an inner arched section 13 merging with a straight entry section 13E whose edges are chamfered. The arched section of recess 13 in the present embodiment is arcuate, but an angular three-sided geometric arch section is also envisaged, as illustrated by the geometric arch 13' shown in Fig. 7. Other arched configurations are also possible.

At its upper end, former 12 is provided with an offset lug 14 which is turned out of the general plane of former 12 so as to lie substantially perpendicular thereto. Lug 14 is received within a slot 15 formed in the nose of handle 8. The orientation and shape of slot 15 is such that, throughout movement of handle 8, it acts on lug 14 in a direction which is parallel to the intended direction of sliding movement of former 12.

A detent and release mechanism is provided for controlling sliding movement of former 12. This mechanism includes a pair of first parts 16 mounted on the upper edge of handle 8 (as viewed in Fig. 2) through the intermediary of a resilient support plate 17. Plate 17 and the first parts 16 are moulded integrally with handle 8. The detent and release mechanism also includes a pair of second parts 18 which are moulded integrally with the respective housing parts 1 and 2 so as to extend inwardly from the side walls thereof.

Each of first parts 16 includes a ribbed portion 16a whose ribs extend laterally relative to the direction of movement of the part during pivotal movement of the handle. Each first part 16 further includes a chamfered leading edge 16b and a portion 16c which is inclined with respect to the portion 16a and has a smooth surface. The portion 16c is inclined at an angle of about 45° to the general direction of movement of first part 16 and is disposed on the opposite side of first part 16 to the associated second part 18 when handle 8 is in the position shown in full line in fig. 2; i.e., the inoperative position of former 12.

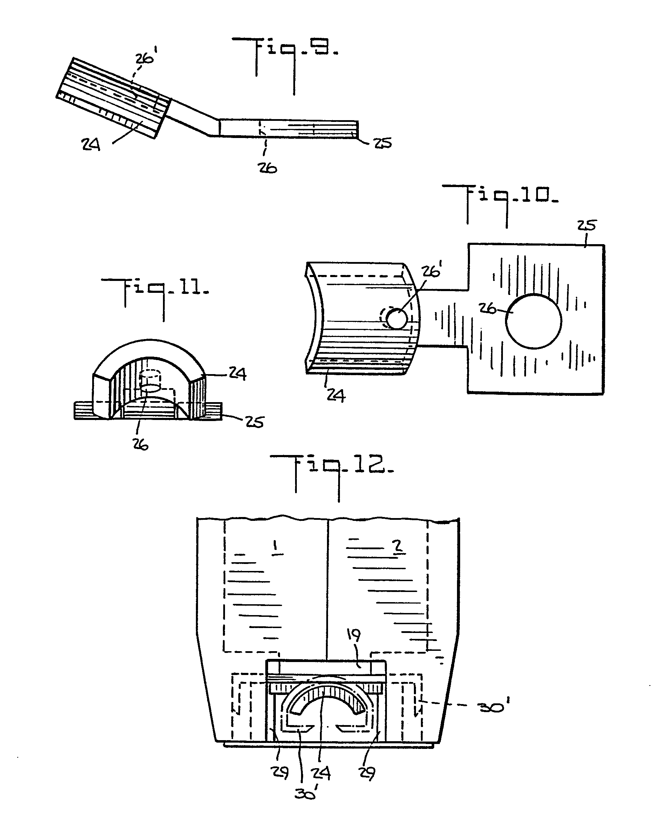

Each of second parts 18 includes a ribbed portion 18a whose ribs extend laterally relative to the direction of movement of first part 16. Each second part 18 further includes a chamfered leading edge 18b and a portion 18c which is inclined with respect to the portion 18a and which has a smooth surface. Portion 18c is inclined at an angle of about 45° to the general direction of movement of first part 16 and is disposed on the opposite side of second part 18 to the ribbed portion 18a. Portion 18c is disposed in the path of movement of portion 16c with the plate 17 in an unflexed condition (as illustrated in Fig. 2). As can be seen from Fig. 3, first parts 16 are supported only at their mutually inwardly-facing edge, whereas second parts 18 are supported only at their mutually outwardly-facing edges.

Provided in the lower region of the housing and adjacent to an outlet 19 therein is a staple indexer 20. The indexer, as best seen in Fig. 8, comprises a plastics rail 20a and a steel mandrel 20b. Plastic rail 20a extends into a magazine 21 (see Fig. 2) containing surgical closure elements in the form of a row of staples (not shown) which straddle the rail. The rail is joined to the housing by ultrasonic welding.

As shown in Figs. 9 and 10, mandrel 20b has an arched position 24 and a plate-like anchor position 25, both portions having a locating hole (26 and 26'). The plastics rail 20a is formed by injection moulding, one end of rail 20a being moulded around anchor position 25 and part of the arched portion 24 of the mandrel so that the plastics material enters the locating holes 26 and 26' and serves to securely retain mandrel 20b in its position at the front end of the rail.

The arched portion 24 of the mandrel is of a shape and size complementary to the arched recess 13 in former 12 (see Fig. 6). Mandrel 20b has a free end opposite anchor portion 25 which is embedded in the end 28 of rail 20a. The location for forming the staples lies between the free end of the mandrel and end 28 of rail 20a. End 29 of rail 20a is configured to form a pair of abutment surfaces 29 on either side of mandrel 20b where it joins rail 20a. These abutment surfaces 29 extend generally perpendicularly to the mandrel in a downward direction (i.e., on an opposite side of the mandrel to the former 12) and are adjacent the location for forming staples.

Operation:

In operation, former 12 acts upon the cross-piece of the staple on the mandrel and causes the side limbs thereof to abut against abutment surfaces 29 as it is being bent downwardly by former 12. The abutment of the staples against surfaces 29 during forming prevents the formation of a twisted staple.

The spring-biased pusher shoe 22 serves to urge the staples along rail 20a toward mandrel 20b at the forming location of the staples which is disposed in the path of movement of former 12. Abutment surfaces 29 are spaced from the housing parts 1 and 2 by a gap and lie at an angle of about $2^{\circ}$ to the inner face of these parts, thus providing a tapered gap which is wider at the top than at the bottom between abutment surfaces 29 and housing parts 1 and 2. The minimum thickness of the gap is marginally less than the thickness of a staple.

In operation, as shown in Fig. 12, a staple 30 will be retained on the mandrel if the handle is released when the staple is only partially formed because the depending limbs of the staple will be frictionally held between abutment surfaces 29 and the housing parts 1 and 2. When the staple is completely shaped to assume form 30', it is no longer held between the abutment surfaces and the housing and is of a shape capable of passing through outlet 19. This feature allows partial formation of the staple 30 prior to placing it over the skin incision and completing the closure without the danger of the partially formed staple dropping out of the device.

The surgical stapling device is positioned on the skin of the patient so that outlet 19 lies across the incision to be closed. Then by squeezing handle 8 against the action of the spring arm 11, this causes former 12 to be driven downwardly so that its lower end passes the mandrel 20b which is accommodated in the arched recess 13 at this stage. During squeezing of handle 8, the first parts 16 of the detent mechanism are moved toward the respective second parts 18 until leading edges 16b and 18b come into mutual engagement. Further movement of handle 8 causes leading edges 16b to ride over edges 18b, this being permitted by resilient flexing of support plate 17, so that the ribbed portions 16a and 18a come into mutual engagement.

The angles of inclination of the sides of the ribs on the ribbed portions 16a and 18a are chosen so that the ribs on the ribbed portions 16a can ride over the ribs of the ribbed portions 18c when handle 8 is squeezed. However, upon release of handle 8, movement of handle 8 and thus former 12 in the opposite direction under the action of spring arm 11 is prevented by interlocking the ribs of the ribbed portions 16a and 18a.

This interlocking action can occur at a plurality of positions intermediate the limit position of movement of former 12 because of a plurality of ribs are provided. Thus, close control over various stages in the formation of a staple can be exercised by the operator because the mechanism can hold handle 8 until the staple is finally closed. In this condition, first parts 16 have moved beyond the respective second parts 18 and, upon release of handle 8, spring 11 returns handle 8 and former 12 back to the original position (as shown in Fig. 2) because the plate 17 flexes back to its original position and brings the smooth portions 16c and 18c into alignment so that the parts 15 are free to ride back over the respective parts 18.

During the staple closing stage, the points of the staple enter the skin on opposite sides of the incision and the limbs bend inwardly toward each other so that the incision in the skin is closed thereby. It will be appreciated that deformation of the cross-piece of the staple occurs as a result of contact with mandrel 20b and with portions of the lower end of the former 12 on opposite sides of the arched recess 13.

The final arched shape of the cross-piece causes an arched air gap to exist between the skin incisions and the cross-piece of the staple even when the wound swells during healing. The portion of handle 8 under slot 15 abuts the upper surfaces of boss 14 to limit inward movement of the handle, thereby limiting movement of former 12 in the direction of mandrel 20b. This movement is arranged so that the free end of mandrel 20b is not so securely trapped between the staple and the skin that it cannot be removed when the staple has been fixed in position. The position of handle 8 at this stage is indicated in chain dot line in Fig. 2.

When handle 8 has been released, as described above, former 12 is clear of mandrel 20b so as to permit pusher shoe 22 to advance the row of staples to place a fresh staple in the path of movement of former 12. In order to give the operator an indication of the number of staples left in the magazine, a slot 23 is provided in housing part 2 adjacent magazine 27. Pusher shoe 22, which can be viewed through this slot 23, is preferably of a contrasting colour to that of housing part 2. In this way, the position of shoe 22 can be readily seen, and the operator then knows the extent to which the staple supply has been depleted.

While there has been shown and described a preferred embodiment of the invention, it will be appreciated that many changes and modifications may be made therein without, however, departing from the essential spirit thereof. Thus, for example, a stapler may be made without the detent and release mechanism. And scale indicia may be inscribed along the edge of the housing under slot 23 so that the surgeon can use this scale to space the staples applied along the incision line.

CLAIMS:

1. A surgical stapling device adapted for use with staples comprising a pair of parallel limbs bridged by a straight cross-piece and arranged to deform the cross-piece during implantation so as to create an arched gap between the underside of the cross-piece and the skin surface.

2. A skin-closure device comprising a housing; a handle movably mounted relative to the housing; a former mounted in the housing so as to be movable upon movement of the handle in one direction from in inoperative limit position to an operative limit position; a mandrel disposed adjacent an outlet opening in the housing; means for supplying skin-closure staples to a location on the mandrel which is in the path of movement of the former in said one direction; said former and said mandrel being shaped so that when the device is used to close an incision in the skin, the cross-piece of the staple is arched above the incision so as to ensure that there is an air gap provided between the incision and the closure element.

3. A device as claimed in claim 2 wherein the cross-piece of the staple is arcuately arched.

4. A device as claimed in claim 2 or 3 wherein the mandrel has an anchor portion which is embedded in a plastics rail which forms part of the means for supplying the staples.

5. A device as claimed in claim 2 or 3 or 4 further comprising means for inhibiting twisting of the staple during formation whilst allowing removal of the mandrel of the forming device after the staple is in position in the skin.

6. A device as claimed in claim 5 wherein the mandrel has a free end on the opposite side of said location to the staple supplying means such as to enable the mandrel to be detached from a formed staple in use; and abutment means are disposed between said location and said staple supply means and extending away from the mandrel on the opposite side thereof to said former so as to be abutted by a staple during forming thereof whereby twisting of the staple during forming is inhibited.

7. A device as claimed in claim 6 wherein the abutment means is defined by an end surface of a rail which forms part of the means for supplying skin-closure staples in which said part of the mandrel is preferably embedded.

8. A device as claimed in claim 7 wherein the end of the rail forming part of the means for supplying skin-closure staples is spaced from the housing by a distance which is marginally less than the thickness of a staple such that the part-formed staple will be retained in position on the mandrel by friction between the staple and the housing and end of the rail, the mandrel being capable of being removed when the staple is fully formed as the staple is then of a shape to pass through the opening in the housing.

9. A surgical stapling device for forming and implanting a staple having a pair of parallel limbs bridged by a straight cross-piece into the skin of a patient undergoing a surgical procedure, the device comprising: a housing having an outlet; a rail disposed in the housing and adapted to have a row of staples straddled thereon and a metal mandrel projecting from the front end of the rail into the outlet; pusher means on said rail to advance said row toward said mandrel to cause the foremost staple to be deposited on the mandrel, the mandrel having an arched shape and being flanked on opposite sides by a pair of abutments on the front end of the rail; a plate-like former disposed in the housing, the leading edge of the former having a recess therein leading to an arched section shaped generally complementarily to the shape of the mandrel, said former being normally retracted from the mandrel to assume an integrated position; and actuating means operatively coupled to the former to advance the former from its inoperative position to slide along the abutments at the front end of the rail to an operative position, in the course of which the mandrel is received within the recess in the former and the limbs of the staple engaged by the leading edge of the former are extended from the outlet to a degree sufficient to penetrate the skin of a patient on opposite sides of an incision to be closed, the limbs being caused to bend inwardly by the co-acting former and mandrel toward each other to close the incision, and the cross-piece being bent to create an arched air gap above the skin to accommodate post-operative swelling.

10. A device as claimed in claim 9, wherein said rail is formed of plastics material and said mandrel is formed of steel, and wherein said mandrel is provided with a blade-like rear anchor which is embedded in the front end of the plastic rail.

11. A device as claimed in claim 9 or 10, wherein said pusher means is a spring-biased shoe which rides on said rail.

12. A device as claimed in claim 9 or 10 or 11, wherein the trailing end of the former is bent to form a lug for advancing the former.

13. A device as claimed in claim 12, wherein said actuating means is constituted by a handle which is pivoted to the housing and is biased to normally swing outwardly therefrom, said handle having a slot in its nose in which the former lug is received, whereby when the handle is swung inwardly toward the housing, the former is advanced thereby.

14. A device as claimed in any of claims 9 to 13, wherein said mandrel arch has an arcuate form.

15. A device as claimed in any of claims 9 to 13, wherein said mandrel arch has a three-sided geometric form.

16. A device as claimed in any of claims 9 to 15, wherein said housing is formed by a pair of complementary moulded plastics pieces.

17. A skin-closure device comprising a housing; a handle pivotally mounted relative to the housing; a former mounted in the housing so as to be movable upon pivotal movement of the handle in one direction from an inoperative limit position to an operative limit position; a first resilient means biasing the former in the opposite direction; a mandrel disposed adjacent an outlet opening in the housing; means for supplying closure elements to a location on the mandrel which is in the path of movement of the former in said one direction; and a detent and release mechanism which retains said former in at least one intermediate position against movement in said opposite direction when the handle is released during movement of said former in said one direction but which permits said former to be moved in said opposite direction into its inoperative limit position by said first resilient

0168244

-7-

means upon release of the handle when the former has reached its operative limit position.

18. A device as claimed in claim 17 wherein the first resilient means is arranged to act on, and is most preferably integral with, the handle and thereby on said former and, preferably also, said detent and release mechanism is similarly arranged.

19. A device as claimed in claim 17 or 18 wherein the detent and release mechanism includes a first part which is mounted for movement with the handle, a second part which is fixed relative to said housing, and a second resilient means biasing at least one of said first and second parts so that the second part is arranged in the path of movement of the first part, each of said first and second parts having a ribbed surface portion and a relatively smooth surface portion which are mutually inclined, the ribbed surface portions of the first and second parts being arranged (a) so that, in said at least one intermediate position of said former, the ribbed surface portions are urged into mutual engagement by said second resilient means and (b) so that they are out of mutual engagement when said former is in said operative limit position whereby

-8-

said second resilient means effects relative movement of the first and second parts to enable the relatively smooth surface portions of the first and second parts to come into mutual contact after subsequent release of the handle and thereby permit relatively unrestricted movement of the first part past the second part during movement of said former from its operative limit position to its inoperative position under the action of the first resilient means.

20. A skin-closure device comprising a housing; a handle movably mounted relative to the housing; a former mounted in the housing so as to be movable upon movement of the handle in one direction from an inoperative limit position to an operative limit position; a mandrel disposed adjacent an outlet opening in the housing; means for supplying skin-closure staples to a location on the mandrel which is in the path of movement of the former in said one direction, the mandrel having a free end on the opposite side of said location to the staple supplying means such as to enable the mandrel to be detached from a formed staple in use; and abutment means disposed between said location and said staple supply means and extending away from the mandrel on the opposite side thereof to said former so as to be abutted by a staple during forming thereof whereby twisting of the staple during forming is prevented.

Fig. 1.

Fig. 2.

Fig. 4.

Fig. 5.

1/3

0168244

Fig. 3.

Fig. 6.

Fig. 7.

Fig. 8.

Fig.9.

24    26'    26    25

Fig.11.

Fig.10.

24    26'    26    25

24    25    26

Fig.12.

1    2

19

30'

29    24    29

30'